**Europäisches Patentamt**

**European Patent Office**

**Office européen des brevets**

(19)

(11) Publication number: **0 112 183**
**A2**

(12) # EUROPEAN PATENT APPLICATION

(21) Application number: **83307674.8**

(22) Date of filing: **16.12.83**

(51) Int. Cl.³: **A 61 J 3/07**

(30) Priority: **20.12.82 US 451573**

(43) Date of publication of application:
**27.06.84 Bulletin 84/26**

(84) Designated Contracting States:
**AT BE CH DE FR GB IT LI LU NL SE**

(71) Applicant: **WARNER-LAMBERT COMPANY**
**201 Tabor Road**
**Morris Plains New Jersey 07950(US)**

(72) Inventor: **Wittwer, Fritz**
**Bündtenstrasse 11**
**CH-4411 Lupsingen(CH)**

(74) Representative: **Jones, Michael Raymond et al,**
**HASELTINE LAKE & CO. 28 Southampton Buildings**
**Chancery Lane**
**London WC2A 1AT(GB)**

(54) A method of providing a seal on a capsule.

(57) A method is disclosed for providing a seal on an edible capsule comprising hard shell, coaxially aligned cap and body parts which are telescopically joined thereby defining a junction seam, the capsule containing pharmaceutical ingredients, which method is for the purpose of rendering such a capsule tamper-resistant and tamper-evident, the method comprising adhesively attaching a frangible, edible label over at least a portion of the seam, which label is adapted to cover regions either side of the seam in order that the label fractures upon displacement in any direction of the capsule parts with respect to each other.

EP 0 112 183 A2

-1-

A METHOD OF PROVIDING A SEAL ON A CAPSULE

This invention relates to a method of providing a seal on an edible capsule to render the capsule tamper-resistant and tamper-evident; and to a capsule provided with such a seal by the method.

The capsules which are to be sealed by the present invention are well known and have been in broad use for many years. Such capsules are generally prepared from an edible natural substance such as gelatin, and comprise hard shell, coaxially aligned cap and body parts which are telescopically joined, each part having one end thereof closed, so that upon co-axial joining, the capsule formed thereby is capable of holding a quantity of material. Generally, such capsules are utilised in the pharmaceutical and food industries, to hold edible and pharmaceutically active materials such as medicines, vitamin preparations, and other edibles both solids and liquid. Generally, the materials from which the capsules are prepared are hydrophilic, and thereby adapted to dissolve in the intestine after ingestion.

One of the difficulties that has long been encountered in the use of such capsules hereinbefore described, has been the ability and occasional tendency for the cap and body parts to disengage, whereby the contents of the capsule escape and are lost.

The problem of the disengagement of the capsule parts from each other has recently become more acute, in view of the well publicised deliberate disassembly of certain capsules containing medicaments and the inclusion therein of certain posions such as cyanide. This deliberate act was successfully accomplished because the capsules were not sealed and, upon disassembly and subsequent reassembly, gave no evidence of their tampering. That is, the slip-fit engagement between the capsule parts is easily disrupted allowing the capsule parts to be separated, so that a small but lethal quantitiy of poison or other disruptive agent may be inserted therein.

The events described above have spurred a renewed interest by the industry and the public to develop methods and associated apparatus to render such capsules tamper-resistant by the placement, on a capsule, of appropriate means which indicate when tempering of a capsule has occurred. One such approach to this problem is disclosed in U.S. Patent No. 1861047 wherein a circular band of gelatin, which subsequently hardens, is disposed about the seam that occurs between the respective capsule parts. The hardened gelatin band is presumed to indicate when the capsule parts have been separated and to thereby offer an indication that tampering of the capsule has occurred.

The procedure outlined in U.S. Patent No.

1861047 and the capsules treated thereby have been found to be deficient, as it is possible to separate the body part from the cap part, modify the contents thereof and thereafter to reassemble the capsule from the cap and body parts and reband the rejoined capsule so that there is no indication that tampering has occurred.

Further, while it is desirable to render the capsules tamper-resistant, it is equally desirable to render such capsules tamper-evident, i.e. capable of offering a visual indication of any disassembly, adulteration and reassembly. The need for a tamper-evident seal is far greater, as it is extremely difficult to ensure that each capsule will be properly sealed so as to be tamper-resistant.

According to the first aspect of the present invention there is provided a method of providing a seal on an edible capsule comprising hard shell, coaxially aligned cap and body parts which are telescopically joined thereby defining a junction seam, the capsule containing pharmaceutical ingredients, which method is for the purpose of rendering such a capsule tamper-resistant and tamper-evident, the method comprising adhesively attaching a frangible, edible label over at least a portion of the seam, which label is adapted to cover regions either side of the seam in order that the label fractures upon displacement in any direction of the capsule parts with respect to each other.

Thus, even the slightest mechanical force will cause the label to fracture along a line coincident with the seam, and the resulting fracture will be incapable of obscuring and/or repair.

In one embodiment of the first aspect of

0112183

the present invention, the label is prepared as a film, for instance by casting or extrusion. This film may be prepared by casting an edible film-forming material against a flat moulding surface.

The cast film may subsequently be dried and thereafter cut to form a label.

In this embodiment of the present invention the labels may be prepared from a number of pharmaceutically acceptable, edible film-forming materials such as:

gelatin;

natural proteins, preferably collagen;

cellulose;

cellulose derivatives;

carbohydrates, preferably starches or modified starches;

vinyl polymers, preferably polyvinyl acetate;

acrylic polymers, preferably polymethacrylic acid, polyacrylic acid or polymethyl methacrylate;

natural gums, preferably gum arabic, gum tragacanth, locust bean gum or guar gum;

and mixtures thereof.

The film-forming material may be prepared in solution using an edible solvent chosen from: water, lower alkyl alcohols having 1-8 carbon atoms, glycols; glycol ethers; lower alkyl ketones having 1-8 carbon atoms; lower alkyl carboxylic acids wherein lower alkyl is defined as ranging from 1-8 carbon atoms, and mixtures thereof.

The film-forming material may contain other additives such as: softeners, colourants, lubricants, anti-oxidants, and mixtures thereof.

In this embodiment of the present invention, the film is initially coated on one side thereof with an edible adhesive compatible with the film and the material constituting the capsule, and the film is thereafter attached to the seam with the edible adjesive positioned therebetween.

The adhesive used in this aspect of the present invention may be chosen from:

a composition comprising a hot melt;

a composition comprising an aqueous solution of a material chosen from: acidic buffers, lower alkyl alcohols having 1-8 carbon atoms, natural proteins, carbohydrates, cellulose derivatives, gums, vinyl polymers, and mixtures thereof;

water and steam, the latter in the instance where it is desired to activate the surface of the label to render it tacky and thereby receptive to the capsule surface; and

cross-linking synthetic monomers, natural and synthetic resins in low boiling point organic solvent solutions, polymer melts, and mixtures thereof.

Alternatively, the adhesives may be pressure-sensitive, and preferably chosen from:

wood rosin derivatives, terpene derivatives, coumarone-indene resins, natural rubbers, synthetic rubbers, acrylic polymers and copolymers, and mixtures thereof.

The adhesives used in this aspect of the present invention may be applied in the heated condition or in the vapourised state, and they may be applied by spraying.

The labels produced in this embodiment of the present invention may bear certain visible indicia, or markings, which may be applied to the labels prior or subsequent to their application to the capsule.

In a second embodiment of the first aspect of the present invention the label is prepared as a hot melt liquid and is applied against the seam while in the heated liquid state, preferably the hot melt being applied from a jet.

In another embodiment the label may be self-adhesive.

According to all three embodiments of the first aspect of the present invention, the seal may be chosen from:

a label comprising a longitudinal strip disposed annularly over at least a portion of the seam, preferably over the entirety of the seam such that the label cooperates with the capsule walls to render the capsule substantially fluid-tight;

a label comprising a longitudinal strip disposed at an angle across the seam, preferably transversely across the seam; and

a label comprising a round patch disposed over the seam along a portion thereof.

The first aspect of the present invention may be practised in a continuous fashion, wherein individual label materials are cast, dried, coated with adhesive and thereafter applied to the assembly capsules. Alternatively, the labels may be cast as continuous sheets which are then punched or otherwise cut to form individual labels for later use.

The first aspect of the present invention may be practised on a variety of different apparatus, including conventional label application machinery. The labels thus applied remain permanently bound to the capsule surfaces, so that even slight dislocation of the capsule parts with respect to each other will cause a fracture to form in the label which will remain permanently evident and incapable of reconstitution.

In a further aspect of the present invention, there is provided a capsule bearing a label, this combination being produced by a method of the first aspect of the present invention.

Capsules sealed in accordance with the first aspect of the present invention exhibit tamper-evident capability as well as a substantial improvement in tamper-resistance. In the instance where the label is prepared from a material that is applied directly to the capsule seam as a hot melt, the nature of this material is such that attempts to reheat the region around the seam after dislocation of the capsule parts with respect to each other, are unsuccessful.

Further, in most instances, the capsule walls appear to lose their crystallinity and corresponding strength as a result of exposure to the temperatures at which the hot melt is applied, so that attempts to separate the cap and body parts of the capsule frequently results in total disintegration thereof.

The first aspect of the present invention may be practised continuously in all of its variant forms, and is therefore capable of high speed, commercial application. As the label may comprise a rounded patch or dot, such as a circle, and may thereby be applied by a direct technique such as jetting of a hot melt, the expense and disruption of encapsulated product manufacture and packaging is minimised. The resulting capsules, however, offer greater security and promote improved user confidence.

The present invention makes it possible to provide a method of sealing capsules comprising hard shell, coaxially aligned cap and body parts which are telescopically joined to render them tamper-evident, by the formation of a seal at the junction seam defined by the capsule parts, which seal serves as a deterrent to disassembly of the capsule.

The method of the present invention can be simply and inexpensively practised, yielding uniformly reliable results.

The present invention makes it possible to provide a capsule seal which is incapable of cosmetic reconstruction after fracture.

The method of the present invention may

-8-

comprise preparing a label, applying an adhesive to one side of the label and therafter applying the label with its adhesive coated surface against the capsule seam.

Alternatively, the label may be prepared and directly applied to the capsule, as in the instance where the label material is a heated liquid that is directed against the capsule's outer wall. In such an instance, it may be unnecessary to utilise the intermediate adhesive, as the label material will possess sufficient adhesive properties to form a durable bond with the outer wall of the capsule.

The labels may be prepared from a variety of edible film-forming materials that are pharmaceutically acceptable. In most instances, these materials are hydrophilic, in that they are water soluble and therefore dissolve readily in the intestinal fluids. Suitable film forming materials may be chosen from: natural proteins, cellulose and its derivatives, carbohydrates, vinyl polymers, acrylic polymers, natural gums and mixtures of these. More particularly, the film-forming material may be chosen from: gelatin, collagen, cellulose, cellulose ethers and esters, modified and unmodified starches, substituted and unsubstituted polyvinyl acetate, polymers and co-polymers of acrylic acid and methacrylic acid, and their salts and esters, natural gums such as gum arabic, gum tragacanth, locust bean gum, guar gum, and mixtures of the above.

Cellulosic materials would include lower alkyl substituted cellulose such as methyl cellulose and ethyl cellulose, as well as salts such as sodium carboxymethyl cellulose. Starches would include, for example, potato starch, wheat starch, maize starch, corn starch, rice starch, pregelatinized starches, dextrins, starch acetates, starch phosphates, and other modified starches.

In the instance where the label is to be preliminarily formed and thereafter applied to the capsule, the film-forming material may be combined with a suitable solvent to facilitate the preparations of solutions, dispersions or emulsions of the aforementioned polymeric materials. Suitable solvents include water; lower alkyl alcohols having 1-8 carbon atoms; glycols, and in particular, lower alkyl glycols and ethers thereof; lower alkyl ketones having 1-8 carbon atoms; lower alkyl esters of alkyl carboxylic acids, wherein lower alkyl is defined as ranging from 1-8 carbon atoms; and mixtures of the same. The solvents may be present in a variety of concentrations, depending upon the viscosity that is desired. For example, a suitable range of solvent is between 1% and 70% by weight of the polymer.

In addition, the solutions, dispersions, or emulsions of the film-forming polymers or materials may include other additives such as softeners. Suitable softeners include, for example, polyols such as glycerol, sorbitol, mannitol; polyglycols such as polyethylene glycol and polypropylene glycol; dialkylphthalates, wherein a lower alkyl such as butyl is utilised; lower alkyl citrates in which the alkyl radical has from 1 to 6 carbon atoms; esters of polyols such as the mono-, di-, and tri-acetates of glycerol; and long chain fatty acids such as ricineoleic acid and esters therof. The softeners are generally present in an amount ranging from 0.1 to 50% by weight based on the polymer solids.

Similarly, colourants may be added, particularly as they render the label more difficult to duplicate in the event of fracture. Suitable colourants include those that are pharmaceutically acceptable, such as: the synthetic dyes defined as Food, Drug and Cosmetic Grade; inorganic oxides such as iron oxide; titanium dioxide; and other known materials. The colourants may be used at concentration ranging from 0.001 to about 10%, and preferably from 0.010 to 5% based upon the weight of the film-forming polymer.

Additionally, ingredients such as lubricants may be added in small quantities, for example, in amounts less than about 10% by weight of the polymer solids. Examples of suitable lubricants include talc and magnesium stearate.

The foregoing materials may be combined and suitable films prepared which may be thereafter cut or punched to form the desired label shape. The preparation of the films may vary in accordance with the art, and may be, for example, accomplished by continuous roller or knife coating, spraying, casting on horizontal flat surfaces . In each instance, the films may be peeled from the forming surface or substrate and thereafter punched or otherwise cut to form the desired label shapes.

An optional post treatment of the label films may be useful, in the instance where, for example, the labels are prepared from a gelatin dispersion, In such an instance, a pharmaceutically acceptable cross-linking agent, for example, formaldehyde, glutaraldehyde, carbodiimides, may be prepared in aqueous solution and sprayed or otherwise applied to the surface of relatively freshly formed gelatin films. This treatment, when compatible with the end use of the capsule to be sealed, has the effect of embrittling the gelating label to enhance frangibility and corresponding tamper-evident capability.

In embodiments of the present method, wherein labels are individually prepared before application to the capsules, the film-forming material described above may be appropriately prepared and films developed with thicknesses ranging, for example, from 10 to 500 microns, and preferably, from to 60 microns. As mentioned earlier, the labels may be punched or cut, to form a variety of suitable shapes, among them rectangular, circular or oval.

For a better understanding of the present invention, and to show how the same may be carried into effect, reference will be made, by way of example, to the accompanying drawings in which:-

Figures 1 to 4 are front perspective views illustrating typical capsules sealed in accordance with the first aspect of the present invention;

Figure 5 is a perspective view of the capsule of Figure 4, illustrating the application of printed indicia to the label;

Figure 6 is a schematic illustration of a label-applying apparatus useful in the present invention; and

Figure 7 is a side sectional view illustrating a fluid-jetting apparatus useful in an alternative embodiment of the present in the present invention.

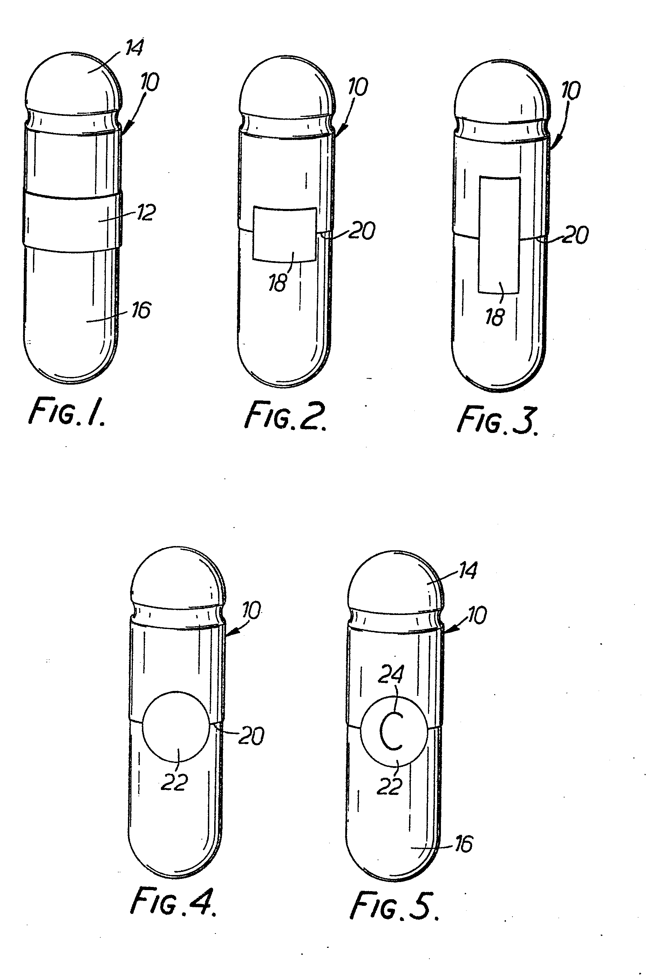

Referring now to Figures 1-4, capsules having labels of various shapes disposed thereon are illustrated. In Figure 1, a capsule 10 is illustrated with a label 12 that is shown extending annularly about the entire capsule, so as to cover the seam (not visible herein) between a cap part 14 and a body part 16.

In this embodiment, the total encirclement of the capsule 10 by the label 12 offers the added effect of tightening the cap part 14 about the body part 16, to enahnce the fluid-retaining capability of the capsule.  Such capsules would therefore be well suited for containing liquids, pastes or the like.

Another label is shown in Figure 2, wherein a finite rectangular strip 18 is placed along the seam 20 of capsule 10.  In the instance where a conventionally sized capsule is concerned, a rectangular strip 18 may, for example, have a length in the range from 5 to 31 mm and a width in the range from 3 to 7 mm.  naturally, these exact dimensions are illustrative only and will vary depending upon discretion and capsule size.

In an alternative applciation, the strip 18 may be applied with its longitudinal dimension transverse to the direction of seam 20, as shown in Figure 3.  A preferred label is shown in Figure 4, wherein a rounded or circular patch 22 is shown.  Exemplary dimensions of patch 22, in the instance of a circular perimeter, are     a diameter in the range from 3 to 10 mm.  Such dimensions are offered as illustrative only.

In the instance where a label is prepared and thereafter applied to the capsule wall, an adhesive composition is preferably applied to one surface of the label.  Suitable adhesives are selected from pharmaceutically acceptable materials, and may comprise water and steam; aqueous acidic buffer solutions; aqueous solutions of lower alcohols; aqueous solutions of natural proteins dissolved in alkaline solutions; aqueous solutions or dispersions of starches; cellulose

-13-

derivatives; carbohydrates, for example gums and sugars; and synthetic polymers, for example, polyvinyl alcohol, polyvinylpyrrolidone.

The adhesives may also include organic solutions of, for example, nitrocellulose, polyvinylacetate, in organic solvents with low boiling points, such as methyl or ethyl acetate, methanol, methylene chloride, and mixtures of these. The adhesives known as "instant glues" are also contemplated, and would include pure monomeric methyl or ethylcyanoacrylates.

In addition to the adhesive materials mentioned above, other materials identified as "hot-melt" materials are contemplated herein, which would be applied by appropriate techniques against the surface of the label. Such "hot-melt" materials would include, for example, hydropolymers such as gelatin, dextrins, natural and synthetic polymers, such as cellulose derivatives, vinyl polymers, including esters and acetals. These materials would, for example, contain in the order of 20% by weight, of water, and would be kept molten at temperatures ranging from 80 to 130°C, at which temperatures they would be applied to the label surface.

In a particular embodiment of the invention, the adhesive composition may be pressure-sensitive. This type of adhesive has particular advantages, in that it simplifies the application of the label to the capsule wall, eliminating the need for the application of either heat or solvents to render the adhesive surface active. Suitable pressure sensitive adhesive compositions include those based on wood rosin, natural or synthetic terpene resins, coumarone-indene resins, natural rubbers, synthetic rubbers such as butadiene-stryrene, isobutylene-isoprene copolymers, polyisobutylene, polyacrylic acids and esters thereof, and copolymers of these materials. As with the other adhesive compositions, the pressure-sensitive adhesives may be applied by any one of numerous well known technieques, for example, dipping, spraying, roller coating. Additional adhesives may be utilised in accordance with the teachings of our copending European Patent Application No. 83305331.7.

The adhesive compositions may include other additives such as softeners, antioxidants and other of the materials recited with respect to the film-forming compositions. Naturally, all of these materials must be pharmaceutically acceptable to find use herein.

The adhesive may be applied to the surface of the label in amounts which vary depending upon the nature of the adhesive and the dimensions of the labels themselves. For example, the adhesive may be applied in amounts in the range from 2 to 10 microlitres per label, in the instance where the labels conform in size to the approximate dimensions set forth earlier herein.

The temperature at which the adhesives may be applied may vary with the specific adhesive. For example, the cyanoacrylate adhesives are utilised at room temperature, while the hot-melt adhesives are preferably utilised at temperatures in the range from 70 to 100°C.

With respect to the hot-melt adhesives, an advantage of these materials, mentioned earlier, is that they offer an increased margin of safety against tampering, by their effect upon the capsule wall. That is, the temperature at which these adhesives are applied causes a local remelting of the capsule wall which enhances the adhesive bond between the label and the

-15-

wall, and also tends to embrittle the wall due
to the rapid cooling thereof that takes places
after the adhesive is applied.   The combined
effect of these events enhances the frangibility
of the capsule wall and this altered property,
in combination with the improved adhesion of
the label increases the likelihood of disintegration
of the capsule in response to a tampering attempt.

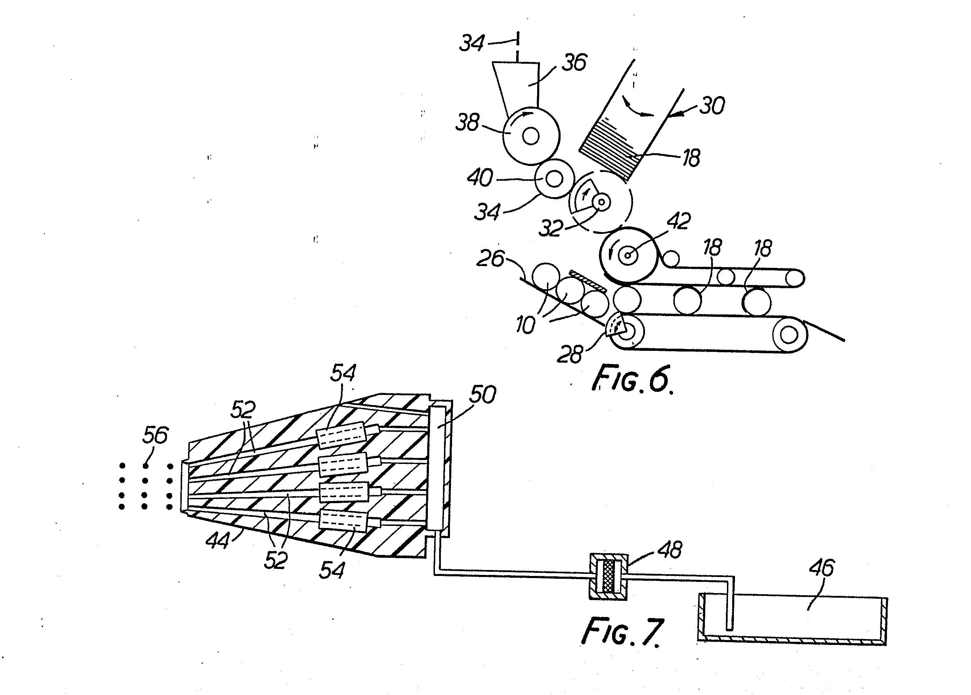

The embodiment of the present method, discussed
above, relates to the sequential preparation
of the label, followed by the application of
an adhesive to a surface of the label, and the
subsequent application of the label to the capsule.
While this procedure may be performed manually,
it is to be understood that automated  operation
of this method is commercially necessary and
desirable.   Referring now to Figure 6, a schematic
diagram of a known label-applying machine is
shown, which may be utilised to put the present
invention into effect.   The machine illustrated
in Figure 6 is commercially available.

Briefly,  the operation of the machine proceeds
as follows.   The capsules 10 enter the machine
via an infeed shute or incline 26 and are sequentially
urged into position for application of the labels
18 by a pusher device 28.   The labels 18 are
extracted from am oscillating magazine 30 by
a label carrier 32 having on its surface a coating
of adhesive 34.   A quantitiy of adhesive 34
is contained within a feed hopper 36 and is
uniformly dispensed upon a roller 38 which in
turn transfers the adhesive layer to an intermediate roller 40, that applies the coating
to the label carrier 32.   Thus, the label carrier
32 extracts a label 18 and rotates to transfer
it to a labelling cylinder 42 which in turn
makes contact with the capsule 10 as shown.

After the label 18 is applied to the surface of the capsule 10, the capsules are non-rotatably transported out of the machine, for packaging or storage.

The machine just described is representative of a variety of automated apparatus useful for the sequential application of labels to capsules. Numerous alternate apparatus may be used to practise the foregoing method, and representative alternatives of them are discussed, though not illustrated. For example, the adhesive may be applied by suitable spray guns directed at labels transferred along a vacuum conveyor or roll, and therafter either heated to dry the adhesive or be directly advanced into contact with the capsule. In a modification of this concept, a vacuum cylinder bearing a regularly spaced plurality of labels may first make contact with a roller coater or other coating device for the dispensing of a quantitiy of adhesive, after which the vacuum conveyor or cylinder may advance into position either for the drying of the adhesive or for the direct application of the adhesive-coated label to a capsule. All of the foregoing techniques, including the application of the adhesive to labels as a hot-melt, may be performed with equipment known in the art, and further discussion thereof is not believed necessary herein.

Further, self-adhesive labels as known in the art may be prepared and used herein. For example, a plurality of such labels may be disposed along a carrier web and would be capable of advancing into position adjacent to consecutive capsules for autogenous transfer to the capsule walls. Such self-adhesive labels would include labels bearing pressure-sensitive adhesives, as well as labels having one of their surfaces sensitized to exhibit an adhesive affinity for the capsule walls.

In an alternate embodiment of the first apsect of the present invention, the label material may be applied directly to the capsule as a hot-melt, or, for example, as an emulsion solution, by a       jetting technique.   Referring now to Figure 7, a representative multi-nozzle assembly is illustrated, which is the subject of European Patent Publication No. 011269 A1. The disclosure of this patent is incorporated herein by reference.

Briefly, with reference to Figure 7, the multi-channelled nozzle assembly 44 draws the fluid from a fluid tank 46, through a conduit with a filter 48 and thence to a fluid distribution chamber 50 where it is introduced into a plurality of nozzle elements 52, each of which has at its proximal end a piezoelectric transducer 54.   Each transducer 54 serves the function of causing the relatively high viscosity incoming fluid to oscillate and thereby to break into droplets such as those illustrated at 56, which may then effectively and rapidly exit the nozzle elements 52.

Thus, by the representive apparatus described in Figure 7, a quantitiy of the film-forming material  suitable for label preparation may be maintained in liquid form and appropriately dispensed with great accuracy against the seam of a capsule, to form the desired label.   This particular technique is useful in the instance where excessive handling of the capsule is undesirable; as contactless application avoids the problems associated with the forces exerted upon the capsule during label application.

In addition to the application of the label to the capsule, imprinting of, for instance, logos and codes may be placed on the visible surface of the label. Such imprinting may be conducted either before the label is applied to the capsule, or after. In the former instance, it is best to utilise conventional techniques such as offset printing, to apply the indicia to the labels, for example, before they are stamped out of a continuous film, or alternatively, just prior to the application of adhesive thereto. In the instance where the labels are applied with a contactless technique as described above, similar contactless printing may be utilised, by a device such as shown in Figure 7 to avoid the unwanted fracture of the capsule. The application of indicia by imprinting provides a further visual characteristic that enhances the tamper-evident capabilities of the seal. As difficult as it is to cosmetically reconstruct a fractured seal, so much more so is the reconstruction fo a fractured logo to assure alignment and continuity. Imprinting, therefore, serves as a valuable additional step that enhances the tamper-evident qualities of the capsule seal prepared by the present invention.

Referring briefly to Figure 5, capsule 10 is shown with a rounded label or patch 22 on which has been imprinted indicia 24. It can be seen that any dislocation of cap part 14 with respect to body part 16, would cause a disturbance of indicia 24 that would be extremely difficult to correct and virtually impossible to obscure.

The present invention is further illustrated by the following Examples. Unless otherwise specified all percentages of materials are expressed in percent by weight.

## EXAMPLE 1

An aqueous gelatin solution with a concentration of 30% by weight, comprising a 50/50 mixture of A 240 Bloom and B 150 Bloom gelatin materials was prepared, containing 2% of titanium dioxide and 0.25% yellow iron oxide, w/w on the basis of dry geletin. This mixture was cast onto a lubricated flat glass plate through a slit of approximately 0.4 mm size, and at a temperature of 55°C. After the setting of the gelatin, the film was dried on the glass plate in a climatic cabinet at 30°C and 30% relative humidity until a water content of about 16% was attained. The films were then peeled off the plates and circular labels having diameters of 5 mm and thickness of about 0.04 mm were punched out for use as labels.

## EXAMPLE 2

A gelatin solution like that in Example 1, but containing 0.2% red iron oxide instead of the yellow iron oxide, was cast onto a lubricated flat glass plate at a temperature of about 55°C. While wet, and after 5 minutes of setting at room temperatrue, the films were dipped into an 8% aqueous glutaraldehyde solution, for 1 minute. The coated films were then dried under ambient room conditions overnight. The thickness of the dry films varied between 0.05 and 0.08 mm. Circular labels of 5 mm diameter were thereafter punched out.

## EXAMPLE 3

A 10% aqueous slurry of pregelatinized maize starch (type Presol D, Roquette national) was heated under stirring until a temperature of 90°C was reached. The homogeneous dispersion appeared like a diluted sol, and was cast onto lubricated glass plates. The resulting films were dried under room conditions and were thereafter peeled off. The films had a thickness of about 0.05 to about 0.07 mm. Transparent labels of 5 mm diameter were punched out.

## EXAMPLE 4

Labels were punched out of commercially available starch paper such as used in confectionery. Three types of starch paper were tested, as follows:

1. Pure maize starch having a thickness of about 0.12 mm.

2. Maize starch, potato starch and vegetable grease.

3. Maize flour and pregelatinized maize starch.

The film thicknesses of formulations 2 and 3 ranged from 0.10 to 0.3 mm.

## EXAMPLE 5

A 20% solution of hydroxypropylmethyl cellulose in water was obtained by stirring slowly at room temperature a mixture of 20 parts of Methocel E5 Premium (having a viscosity of 5 cP ($mN \cdot s/m^2$) at 20°C for a 2% aqueous solution, Colorcon) and 80 parts of water. The resulting clear solution had a viscosity of about 2,000 to 2,500 cP ($mN \cdot s/m^2$) at 20°C. This solution was cast onto lubricated glass plates at 30°C through a slit of 0.5 mm. The films were dried overnight under room conditions and when peeled off had a thickness in the range from 0.04 to 0.065 mm. Rectangular labels were punched out with dimensions of 7 mm by 4 mm.

## EXAMPLE 6

15 Grams of sorbitol, 0.9 grams of Ponceau 5X and 0.16 g of brilliant blue FCF were dissolved in 2.5 litres of hot water, and the resulting solution was added to 520 g of a 15 cP (mN s/m$^2$) type methyl cellulose. This mixture was stored at about 5°C for 24 hours to allow the methyl cellulose to dissolve. At the end of this period, water was added in order to bring the volume to 2.7 litres. The solution was well mixed and allowed to stand for de-bubbling. It was then cast onto lubricated glass plates. The resulting films were dried overnight at 20°C and 40% relative humidity in a climatic cabinet. The dry films were peeled off and circular labels of a 6 mm diameter were punched out, having a film thickness of 0.6 mm.

### EXAMPLE 7

50 Millilitres of a 10% diethyl tartrate solution were prepared in water. 15 Grams of hydroxypropylmethyl cellulose phthalate (HP55F grade-Shin Etsu chemical) was added gradually under magentic stirring. Thereafter, 100 ml of an aqueous 5% Citroflex solution were prepared after which alternative additions fo 20 ml portions of Citroflex solution and 35 g portions of HP55 were added to the initial dispersion under magnetic stirring. Thereafter, the entire solution was de-bubbled under reduced vacuum.

37.5 Millilitres of a 2% Methocel (K 15M grade, Colorcon) solution was prepared in water. To this solution, 0.5 g polypropylene glycol, 7.5 g of Citroflex 2 solution and 5.62 g of pure Citroflex 2 were added in succession and under magnetic stirring. The homogenity of the resulting emulsion was controlled.

The above emulsion was added to the dispersion of HP55 under constant storring. The ratio between the dispersion and the emulsion of Methocel was kept as close as possible to 3.9:1. The resulting dispersion was stored at room temperature overnight. Thereafter films were cast on lubricated glass plates at room temperature, and were thereafter dried for 1 hour at 60°C, whereupon they were peeled off. Labels having diameters of 4 and 6 mm respectively, were punched out of the sheets, and were found to be clear and transparent. The label had a thickness in the range from 0.045 to 0.062 mm.

## EXAMPLE 8

10 Grams of hydroxypropylmethyl cellulose phthalate (HP55F grade) were vigourously mixed with 24 g of ethyl acetate, 6 g of 2-butanol, 11 g of 2-butoxyethanol and 2 g of Tween 80. After total dissolution of the HP55F, a dispersion of 0.2 g titanium dioxide (Arathase grade) in 10 g of water was gradulaly added. A homogeneous emulsion was obtained and with the film casting procedures described earlier herein, white opaque films having thicknesses of about 0.05 mm were obtained, after drying overnight at 50°C. Circular labels having a 5mm diameter were thereafter punched out.

## EXAMPLE 9

7 Grams of cellulose acetate phthalate were mixed with 2.4 g triacetin, 23.5 g ethyl acetate, 8 g of 2-butanol and 8 g of butyl glycol. After the dissolution of the cellulose acetate phthalate at the refluxing temperature of ethylacetate (77°C) a dispersion of 0.2 g titanium dioxide and 0.2 g red iron oxide in 10 ml of ethylacetate was added and homogenously mixed therewith. Utilising the standard film casting procedure described above, pink opaque films having a thickness of 0.04 mm were obtained. The films were dried overnight at 50°C and thereafter circular labels having a 5 mm diameter were punched out.

## EXAMPLE 10

A mixture of 25 g of a copolymer of methylacrylic acid and methylacrylic acid methyl ester (Eudragit L100) and 2.5 g polyethylene glycol 4000, 29 g of acetone and 43.5 g of isopropanol were heated together under reflux until complete dissolution of the methylacrylic copolymer was achieved. The hot mixture was then sprayed onto a Teflon (registered Trade mark) sheet, and after 3 hours of drying at 50°C, a transparent film having a thickness of about 0.05 mm was obtained. Thereafter, circular labels of a 5 mm diameter were punched out.

## EXAMPLE 11

Hard shell gelatin capsules size No. 1, filled with lactose were sealed with individual circular gelatin labels prepared in accordance with Example 1, above. Individual labels were picked up by a labelling roll exerting a vacuum suction. Each label was sequentially advanced past a nozzle having a 0.3 mm orifice through which a quantity of steam was applied against the labels to render them sticky. The labels were then advanced into position adjacent to the capsules, the vacuum in the label roller was broken and application rolls adajcent to the vacuum roll assured the uniform adhesion of the labels to the capsule surfaces by rotating the capsule therepast. The labelled capsules were dried in an air flow, the dried capsules had approximately 16% water in the shell and could not be opened without destroying the label and the capsule.

## EXAMPLE 12

The same capsule, contents and label material as were used for Example 11 were combined herein. In this Example, however, the steam spray system was replaced by a roller coating that transferred 10 microlitres of water at a temperature of 95°C to each label by means of hydrophilic felt rolls. The capsules prepared in accordance with this embodiment exhibited the same resistance to tampering as those produced in Example 9.

## EXAMPLE 13

This Example is similar to Example 12 with the exception that the film forming material comprised starch paper prepared in accordance with Example 4 above. Also, a spray of 5 to 10 microlitres of hot water replaced the steam spary of Example 11 and two labels were applied over the seam of each capsule. The resulting capsules exhibited the same resistance to tampering as those produced in Examples 11 and 12 above.

## EXAMPLE 14

A white opaque film as described in Example 8 was imprinted with a logo by conventional offset printing with a black ink containing shellac EPC Grade and black iron oxide. Round labels of 5 mm diameter were punched out and put into a label magazine of a conventional labelling machine adapted for small cylindrical objects.

A hot phosphate buffer solution having a pH of 6.8 and a temeprature of 60°C was applied to the unprinted side of the labels by a roller coating procedures which was performed while the labels were maitnained in fixed position upon a vacuum roll. The labels were then affixed to the capsule and additional drying thereof was conducted. The resulting capsules could not be opened without destroying the label and the capsule.



CLAIMS:

1. A method of providing a seal on an edible capsule comprising hard shell, coaxially aligned cap and body parts which are telescopically joined thereby defining a junction seam, the capsule containing pharmaceutical ingredients, which method is for the purpose of rendering such a capsule tamper-resistant and tamper-evident, the method comprising adhesively attaching a frangible, edible label over at least a portion of the seam, which label is adapted to cover regions either side of the seam in order that the label fractures upon displacement in any direction of the capsule parts with respect to each other.

2. A method according to Claim 1, wherein the label is prepared as a film, preferably by casting or extrusion, more preferably by casting.

3. A method according to Claim 2, wherein the film is prepared by casting an edible film-forming material against a flat moulding surface.

4. A method according to Claim 3, wherein the film-forming material is chosen from:

gelatin;

natural proteins, preferably collagen;

cellulose;

cellulose derivatives;

carbohydrates, preferably starches or modified starches;

vinyl polymers, preferably polyvinyl acetate;

acrylic polymers, preferably polymethacrylic acid, polyacrylic acid or polymethyl methacrylate;

natural gums, preferably gum arabic, gum tragacanth, locust bean gum or guar gum;

and mixtures thereof.

5.    A method according to Claim 3, or 4, wherein the film-forming material is prepared in solution using an edible solvent chosen from: water, lower alkyl alcohols having 1-8 carbon atoms, glycols; glycol ethers; lower alkyl ketones having 1-8 carbon atoms; lower alkyl carboxylic acids wherein lower alkyl is defined as ranging from 1-8 carbon atoms, and mixtures thereof.

6.    A method according to Claim 3, 4 or 5, wherein the film-forming material further comprises a material chosen from:

softeners, colourants, lubricants, anti-oxidants, and mixtures thereof.

7.    A method according to Claims 2 to 6, wherein the cast film is subsequently dried and thereafter cut to form the label.

8.    A method according to Claims 2 to 7, wherein the film is     initially coated on one side thereof with an edible adhesive compatible with the film and the material constituting the capsule, and the film is thereafter attached to the seam with the edible adhesive positioned therebetween.

9.    A method according to Claim 8, wherein the adhesive is applied by spraying.

10.    A method according to Claim 8 or 9, wherein the adhesive is applied in the heated condition or in the vapourised state.

11.    A method according to Claim 8, 9 or 10 wherein the adhesive is chosen from:

a composition comprising a hot melt;

a composition comprising an aqueous solution of a material chosen from: acidic buffers, lower alkyl alcohols  having 1-8 carbon atoms, natural proteins, carbohydrates, cellulose derivatives, gums, vinyl polymers, and mixtures thereof;

water and steam; and

-27-

cross-linking synthetic monomers, natural and synthetic resins in low boiling point solvent solutions, polymer melts, and mixtures thereof.

12. A method according to Claim 8 or 9, wherein the adhesive is pressure-sensitive, and preferably chosen from:

wood rosin derivatives, terpene derivatives, coumarone-indene resins, natural rubbers, synthetic rubbers, acrylic polymers and copolymers, and mixtures thereof.

13. A method according to any preceding claim, which further comprises printing indicia on the surface of the label, which printing may be prior or subsequent to applying the label to the capsule, and which indicia are visible when the label is affixed to the capsule.

14. A method according to Claim 1, wherein the label is prepared as a hot melt liquid and is applied against the seam while in the heated liquid state, preferably the hot melt being applied from a jet.

15. A method according to Claim 1, wherein the label is self-adhesive.

16. A method according to any preceding claim, wherein the seal is chosen from:

a label comprising a longitudinal strip disposed annularly over at least a portion of the seam, preferably over the entirety of the seam such that the label cooperates with the capsule walls to render the capsule substantially fluid-tight;

a label comprising a longitudinal strip disposed at an angle across the seam, preferably transversely across the seam; and

a label comprising a round patch disposed over the seam along a portion thereof.

17. A capsule bearing a label, whenever produced by a method according to any preceding claim.

FIG.1.

FIG.2.

FIG.3.

FIG.4.

FIG.5.

FIG. 6.

FIG. 7.

0112183